# EUROPEAN PATENT APPLICATION

(11) **EP 3 457 133 A1**
(43) Date of publication of application: **20.03.2019**
(21) Application number: 17796139.8
(22) Date of filing: 09.05.2017
(51) Int. Cl.: G01N 33/53, C07K 14/79

(54) **DIAGNOSTIC MARKER FOR DEMENTIA AND METHOD FOR IDENTIFYING CONTRACTION OF DEMENTIA USING SAID MARKER**

(30) Priority: 10.05.2016 JP 2016094385
(71) Applicant: Hashimoto, Yasuhiro, Fukushima-shi, Fukushima 960-1295 (JP); Fukushima Medical University, Fukushima 960-1295 (JP)
(72) Inventor: HASHIMOTO, Yasuhiro, Fukushima-shi Fukushima 960-1295 (JP); HOSHI, Kyoka, Fukushima-shi Fukushima 960-1295 (JP); ITO, Hiromi, Fukushima-shi Fukushima 960-1295 (JP); HONDA, Takashi, Fukushima-shi Fukushima 960-1295 (JP); YAMAGUCHI, Yoshiki, Wako-shi Saitama 351-0198 (JP); NAGAE, Masamichi, Bunkyo-ku Tokyo 113-0033 (JP)
(74) Representative: J A Kemp
(86) International application number: PCT/JP2017/017543
(87) International publication number: WO 2017/195778

(57) **Abstract**

An object of the present invention is to develop a biomarker for discriminating dementia, particularly, Alzheimer's disease and mild cognitive impairment which can progress to Alzheimer's disease, from other central nervous system diseases, or capable of determining a pathological condition of dementia, and provide a method for diagnosing dementia early and a method for determining a pathological condition of dementia, using the biomarker. A transferrin glycoprotein containing a glycan having at least one mannose at a non-reducing end or a transferrin fragment containing the glycan is used as a diagnostic marker for Alzheimer's disease.

## Description

### Technical Field

The present invention relates to a biomarker for discriminating dementia, particularly, Alzheimer's disease and mild cognitive impairment which can progress to Alzheimer's disease, from other central nervous system diseases, and a method for identifying the contraction of dementia and a method for determining a pathological condition of dementia, using the same.

### Background Art

Alzheimer's disease (hereinafter abbreviated as "AD") is an irreversible progressive central nervous system disease and manifests symptoms such as cognitive dysfunction (dementia) involving memory impairment or thought disorder, behavioral disorder, or personality change. Alzheimer's disease is the most common disease among dementia cases and accounts for approximately 60 to 80% of all dementia patients. Although this disease generally develops in elderly people at age 65 or over, some cases develop in 64-year-old or younger people and are called early-onset Alzheimer's disease.

The number of dementia patients throughout the world is estimated at 35,000,000 people in 2010 and expected to double every 20 years in the future and reach 110,000,000 people in 2050. Approximately 70% of these patients are considered as AD patients. Particularly, in developed countries, the escalation of medical costs, nursing care problems, etc. associated with an increased number of AD patients place increasingly severe economic burden or mental load on the countries or people involved in the patients and are becoming a huge social issue.

Familial (inherited) AD is also a genetic disease caused by mutations in amyloid precursor protein (APP) gene or presenilin 1 (PSEN1) gene. This disease starts with the aggregation and accumulation of amyloid β protein (Aβ) which is a hydrophobic peptide in neurons in the brain. The disease then develops by the hyperphosphorylation and fibrosis of tau protein which is a microtubule protein followed by neuronal destruction and brain atrophy. Even sporadic cases, which occupy most of AD cases, are known to basically develop through a similar pathological process.

Many therapeutic drugs for AD that inhibit the process of development are currently under development on the basis of the mechanism underlying the development of AD. However, most of these therapeutic drugs have not yet exhibited sufficient efficacy in clinical trials. This may not necessarily mean that investigational new drugs are ineffective. Rather, this is probably because clinical trial subject groups are not appropriate. The subject groups undergoing the clinical trials in the past were at a stage where brain lesions due to AD had already progressed and massive neuronal death had already occurred. However, typically, the recovery of the nervous system cannot be expected if treatment is started at this stage. Namely, it is considered that the therapeutic drugs failed to produce an effective therapeutic effect because the time of administration was too late (Non Patent Literature 1). Thus, provided that a therapeutic drug for AD can be administered at an appropriate time for administration, i.e., an early stage when it is possible to circumvent neuronal death, its efficacy must be testable.

Patients who are suspected of having dementia at outpatient clinics for forgetfulness are typically classified, on the basis of examination of, for example, dementia scores, into 3 groups: a normal group, mild cognitive impairment (hereinafter often abbreviated as "MCI"), and AD. From long-term observational study results, it is known that some MCI patients will develop AD in the future, whereas the remaining patients remain as MCI. Namely, some MCI patients can be interpreted as very early AD patients. Such patients with MCI that can transition to AD in the future (herein often referred to as "Alzheimer's disease transition-type mild cognitive impairment (AD transition-type MCI)") rarely have neuronal death. Hence, the AD transition-type MCI patients are highly desirable as a clinical trial subject group for confirming the efficacy of therapeutic drugs for AD. Furthermore, AD transition-type MCI also corresponds to a suitable time for administering therapeutic drugs for AD to obtain an effect of preventing the development of AD or delaying the progression of AD. For these purposes, the development of a method for diagnosing AD early or a method for identifying AD transition-type MCI is indispensable.

Currently, advance in diagnostic imaging technology such as MRI or CT enables the detection of morphological changes or morbid changes, for example, micro-bleeding or tumor, in the brain. However, morphological changes in neurodegenerative diseases such as AD progresses over a long period of 10 to 20 years. Therefore, changes unique to AD, such as ventricular enlargement (brain atrophy), can be captured by imaging only after a massive neuronal death occurs. Thus, it is difficult to diagnose AD early from morphological changes in the brain. Also, morphological changes in the brain are rarely observed in MCI. Therefore, it is difficult to identify AD transition-type MCI from morphological changes in the brain.

In general, AD is definitively diagnosed by pathologically demonstrating amyloid plaques and neurofibrillary tangles in the autopsied brain after death. It was difficult to visualize amyloid plaques as imaging findings in live patients. Recently, a method which involves introducing [¹¹C]Pittsburgh compound (PiB) labelled with ¹¹C which is a positron radioisotope as a radioactive diagnostic imaging drug into the body of a patient and visualizing the accumulation of amyloid protein in the brain by amyloid PET (positron emission tomography), or tau PET using 6-[(3-¹⁸F-fluoro-2-hydroxy)propoxy]-2-(4-methylaminophenyl)quinolone (¹⁸F-THK5117) has been practiced (Non Patent Literature 3). These examination methods can detect AD at a relatively early stage and are therefore effective for testing the early administration of therapeutic drugs for AD in clinical trials. However, these methods are not much suitable as ordinary diagnosis methods because these methods require high cost and long examination time.

Thus, in recent years, there has been a demand for convenient and low-cost biomarkers. For example, methods using causative factors of AD, for early diagnostic markers for AD, have also been developed. Specific examples thereof include a method using hyperphosphorylated tau protein, amyloid β42 (Aβ42) peptide, or cytokine in inflammatory disease, etc. as diagnostic markers for AD (Non Patent Literature 2). However, the cytokine permits highly sensitive measurement, but disadvantageously has poor disease specificity. The phosphorylated tau protein is an excellent diagnostic marker for AD, but is insufficient for the early diagnosis of AD, which is to be performed before circumventing neuronal death, because the appearance of this protein means neuronal death. In addition, the level of the tau protein also increases in dementia such as frontotemporal dementia or progressive supranuclear palsy and therefore, cannot be regarded as a biomarker specific for AD. On the other hand, the Aβ42 peptide has a problem that this peptide exhibits changes only after the disease progresses.

### Citation List

### Non Patent Literature

Non Patent Literature 1: Selkoe D.J., 2012, Science 337: 1488-92
Non Patent Literature 2: Mattsson N et al., JAMA. 2009, 302 (4): 385-93
Non Patent Literature 3: Li Y et al., 2015, J Nucl Med., 56 (2): 270-3

### Summary of Invention

### Technical Problem

An object of the present invention is to develop a biomarker serving as an index for identifying the contraction of AD, or a biomarker capable of determining a pathological condition, such as the degree of progression or curability, of AD, and provide a simple method for diagnosing AD early and method for determining a pathological condition of AD, using the biomarker.

### Solution to Problem

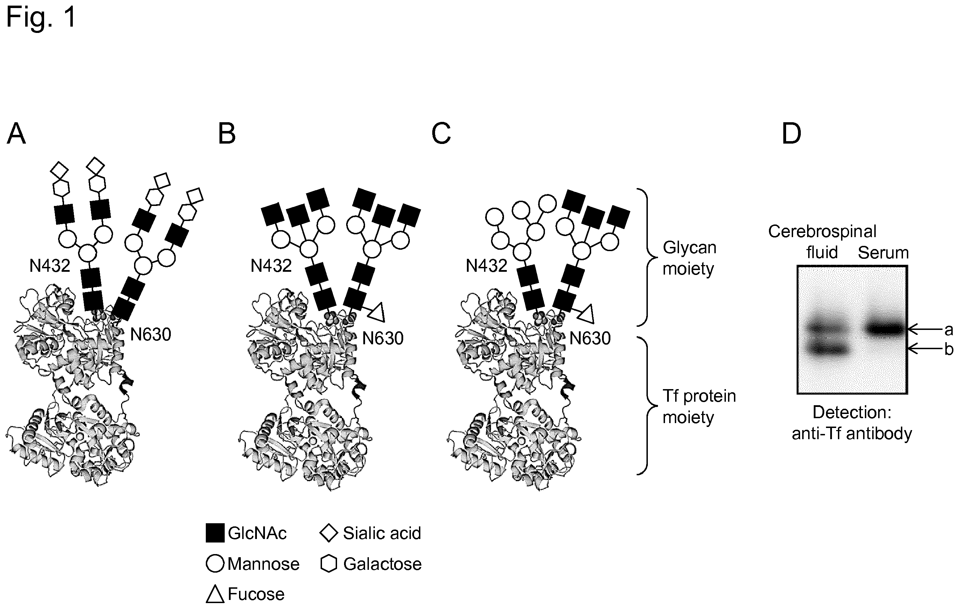

The present inventors has hypothesized that a glycoprotein having a unique glycan structure is present in the central nerve system, and used various antibodies for screening glycoproteins differing in glycan moiety between cerebrospinal fluid and serum. As a result, a transferrin (herein often abbreviated as "Tf") glycoprotein was identified as a glycoprotein having such a feature. The Tf glycoprotein is present as sialylated Tf having sialic acids at non-reducing ends (Figure 1A) in serum. In contrast, two types of Tf glycan isoforms were detected in cerebrospinal fluid, i.e., sialylated Tf and non-sialylated Tf having no sialic acid at a non-reducing end. Furthermore, a sialylated Tf/non-sialylated Tf ratio (Tf index) was found to serve as an index marker for idiopathic normal pressure hydrocephalus (herein often abbreviated as "iNPH") which is dementia based on abnormal metabolism in cerebrospinal fluid (JP Patent Publication (Kokai) No. 2010-121980 A (2010); and Futakawa et al., 2012, Neurobiol Aging, 33: 1807-15).

The present inventors conducted further studies on the basis of the findings described above and consequently found that non-sialylated Tf previously considered to be a single type of isoform includes two subgroups (subgroup 1 and subgroup 2) (Nagae et al., 2014, Glycobiology, 24, 693-702).

As shown in Figure 1, human Tf contains a glycan attached to asparagine residues at positions 432 (N432) and 630 (N630) when the initiator methionine is defined as position 1. In subgroup 1 of non-sialylated Tf (Figure 1B), the glycan at N630 has N-acetylglucosamine (herein also abbreviated as "GlcNAc") at non-reducing ends and is also modified with bisect GlcNAc and core fucose. The glycan at N432 also has GlcNAc at non-reducing ends, but is modified with only bisect GlcNAc without core fucose. On the other hand, subgroup 2 (Figure 1C), which has been newly found by the present inventors, the glycan at N630 is the same as in subgroup 1, whereas the glycan at N432 has mannose (herein also abbreviated as "Man") at non-reducing ends.

The present inventors have found this time that: Tf subgroup 2 containing a glycan having mannose at a non-reducing end serves as a diagnostic marker for AD and MCI; Tf subgroup 2 present in cerebrospinal fluid is quantifiable by using a lectin or an antibody; and the amount thereof correlates with the degree of progression of AD or MCI, reaching the completion of the present invention.

The present invention is based on the findings described above and provides the following.
(1) A diagnostic marker for AD consisting of a Tf glycoprotein containing a glycan having at least one mannose at a non-reducing end or a Tf fragment containing the glycan.
(2) The diagnostic marker for AD according to (1), wherein the glycan is attached to an asparagine residue at position 432 of Tf protein consisting of the amino acid sequence represented by SEQ ID NO: 1.
(3) The diagnostic marker for AD according to (1) or (2), wherein the diagnostic marker is for the identification of early AD or AD transition-type MCI.
(4) The diagnostic marker for AD according to (1) or (2), wherein the diagnostic marker is for the differentiation of AD from other cases of dementia.
(5) A diagnostic kit for AD for detecting a diagnostic marker for AD according to any of (1) to (4), the diagnostic kit comprising a Man binding molecule and a Tf binding molecule.
(6) The diagnostic kit according to (5), wherein the Man binding molecule is selected from the group consisting of a mannose binding lectin, an anti-mannose antibody, and an aptamer against mannose.
(7) The diagnostic kit according to (6), wherein the mannose binding lectin is UDA lectin or BC2L-A lectin.
(8) The diagnostic kit according to any of (5) to (7), wherein the Tf binding molecule is an anti-Tf antibody and/or an aptamer against Tf protein.
(9) A method for identifying the contraction of AD, the method comprising: a measurement step of measuring an amount of a diagnostic marker for AD according to (1) or (2) present in a predetermined amount of a body fluid obtained from each of a test subject and a control group, using a Man binding molecule and a Tf binding molecule to obtain measurement values; and a comparison and identification step of comparing the measurement values of the test subject and the control group obtained in the measurement step to identify the test subject as having AD when the measurement value of the diagnostic marker for AD is significantly higher in the test subject.
(10) The method for identifying the contraction according to (9), wherein the control group is a normal group.
(11) The method for identifying the contraction according to (9), wherein the control group is an early AD group.
(12) The method for identifying the contraction according to (9) or (10), wherein the test subject has a central nervous system disease.
(13) A method for identifying AD transition-type MCI, the method comprising: a measurement step of measuring an amount of a diagnostic marker for AD according to (1) or (2) present in a predetermined amount of a body fluid obtained from each of a test subject and a control group, using a Man binding molecule and a Tf binding molecule to obtain measurement values; and a comparison and identification step of comparing the measurement values of the diagnostic marker for AD in the test subject and the control group obtained in the measurement step to identify the test subject as having AD transition-type MCI when the measurement value of the diagnostic marker for AD is significantly higher in the test subject, wherein the test subject has MCI.

The present specification incorporates the contents disclosed in Japanese Patent Application No. 2016-094385, to which the present application claims priority.

### Advantageous Effects of Invention

According to the diagnostic marker for AD of the present invention, the contraction or potential contraction of AD can be diagnosed, or a pathological condition of AD can be determined.

According to the diagnostic kit for AD of the present invention, the diagnostic marker for AD can be detected easily and at relatively low cost, whereby the contraction or potential contraction of AD in a test subject who is a sample provider can be identified, and/or a pathological condition of AD can be determined.

According to the method for identifying the contraction of AD according to the present invention, the contraction of AD or the potential contraction of AD in a test subject, particularly, a patient having some central nervous system disease, can be identified. Also, a pathological condition of AD present therein can be determined.

According to the method for identifying AD transition-type MCI according to the present invention, an AD transition-type MCI patient having the potential to transition to AD can be identified easily and at high accuracy among patients having MCI.

### Brief Description of Drawings

Figures 1A to 1C are schematic diagrams showing the structures of three Tf glycan isoforms of a Tf glycoprotein present in cerebrospinal fluid. Figure 1A shows a terminal Sia-Tf glycoprotein (sialylated Tf) having two glycans terminating in non-reducing end sialic acid. Figure 1B shows a terminal GlcNAc-Tf glycoprotein (non-sialylated Tf subgroup 1) having two glycans terminating in non-reducing end GlcNAc. Figure 1C shows a terminal Man-Tf glycoprotein (non-sialylated Tf subgroup 2) having one each of a glycan terminating in non-reducing end Man and a glycan terminating in non-reducing end GlcNAc. Figure ID is a diagram showing results of Western blot on cerebrospinal fluid and serum using an anti-Tf antibody. The band "a" detected in the cerebrospinal fluid and the serum corresponds to the terminal Sia-Tf glycoprotein. On the other hand, the band "b" detected only in the cerebrospinal fluid appears to be one Tf glycoprotein, but includes two Tf glycoproteins (subgroups 1 and 2): the terminal GlcNAc-Tf glycoprotein and the terminal Man-Tf glycoprotein.

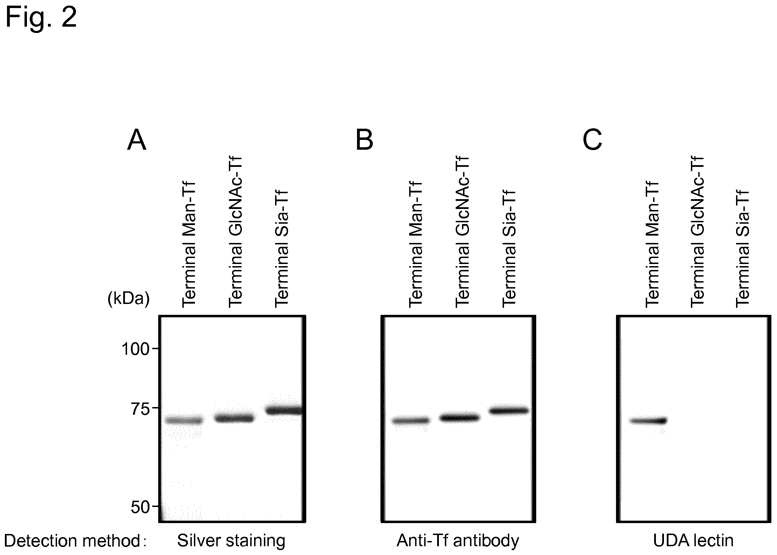

Figure 2 is a diagram showing the glycan binding specificity of UDA lectin. Figure 2A shows silver staining after electrophoresis of each Tf glycoprotein preparation of terminal Man-Tf, terminal GlcNAc-Tf and terminal Sia-Tf. Figure 2B shows an immunoblot using an anti-Tf antibody. Figure 2C shows lectin blot using UDA lectin.

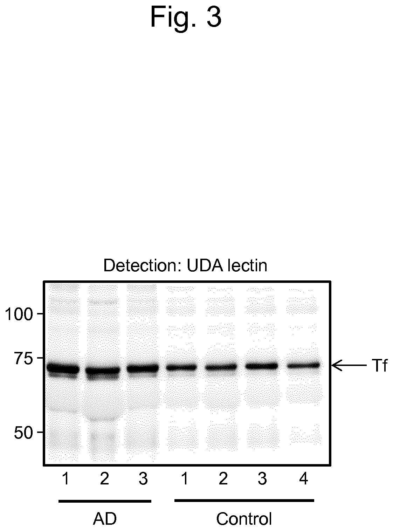

Figure 3 shows results of analyzing Man-Tf in cerebrospinal fluid obtained from an AD group (n = 3) and a disease control group (n = 4), by UDA lectin blot.

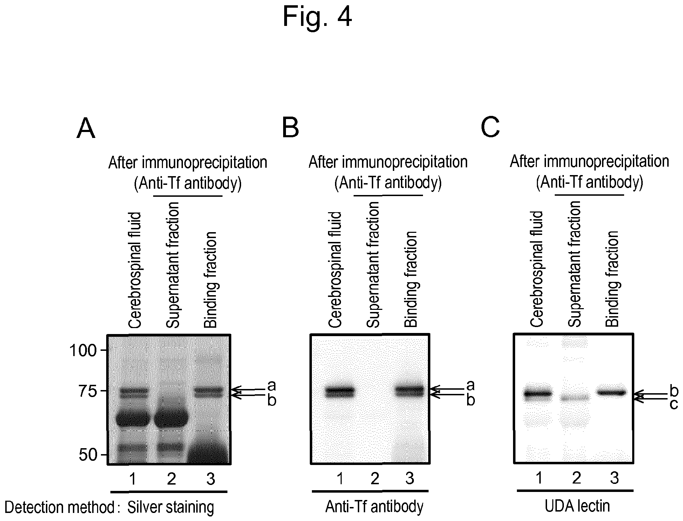

Figure 4 shows results of immunoprecipitation reaction of Tf in cerebrospinal fluid using an anti-Tf antibody. Figure 4A shows results of detecting, by silver staining, samples after electrophoresis of a supernatant fraction (non-binding fraction) after reaction of anti-Tf antibody-bound beads with cerebrospinal fluid, a binding fraction (eluted fraction) with anti-Tf antibody-bound beads, and the starting material (crude cerebrospinal fluid). Figure 4B shows results of detecting these samples by immunoblot using an anti-Tf antibody. Figure 4C shows results of detecting these samples by UDA lectin blot. The band "a" corresponds to terminal Sia-Tf. The band "b" corresponds to terminal Man-Tf/terminal GlcNAc-Tf (terminal Man-Tf in Figure 4C). The band "c" corresponds to a Man glycoprotein (GP-X) other than terminal Man-Tf.

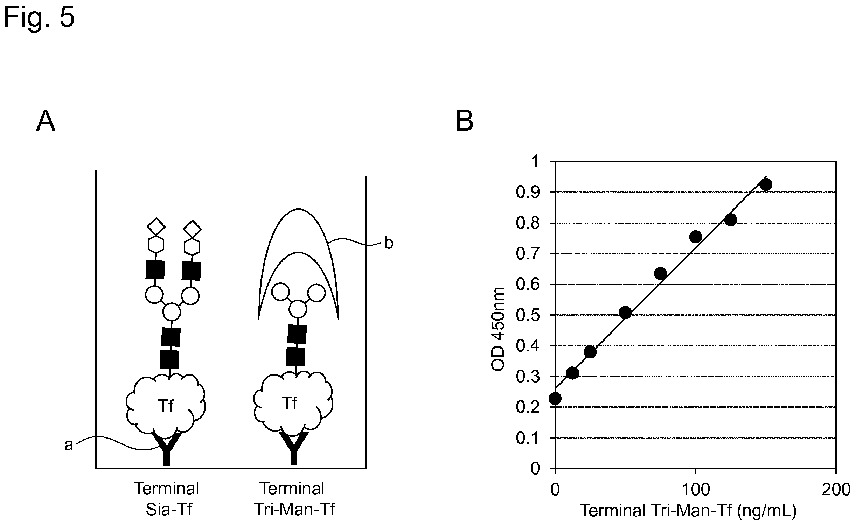

Figure 5A is a diagram of measurement principles of UDA lectin/antibody sandwich ELISA for detecting terminal Man-Tf. In the drawing, "a" indicates an anti-Tf antibody serving as a capturing antibody. "b" indicates UDA lectin. Figure 5B shows a calibration curve when terminal Man-Tf (terminal Tri-Man-Tf) was used. The anti-Tf antibody was immobilized on a microtiter plate, and free Tf was captured. Terminal Man-Tf was detected and quantified with UDA lectin. Terminal Sia-Tf does not bind to UDA lectin and therefore, is not detected. For the calibration curve, terminal Tri-Man-Tf was used as a standard.

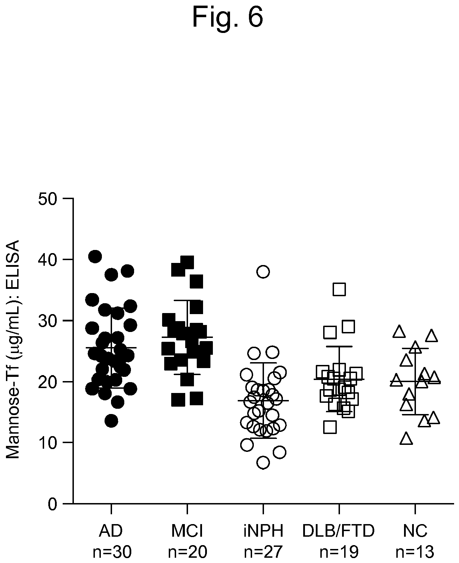

Figure 6 shows results of quantifying terminal Man-Tf in cerebrospinal fluid by UDA lectin/antibody sandwich ELISA for 5 groups: an Alzheimer's disease (AD) group, a mild cognitive impairment (MCI) group, an idiopathic normal pressure hydrocephalus (iNPH) group, a dementia with Lewy bodies/frontotemporal dementia (DLB/FTD) group, and a normal control (NC) group.

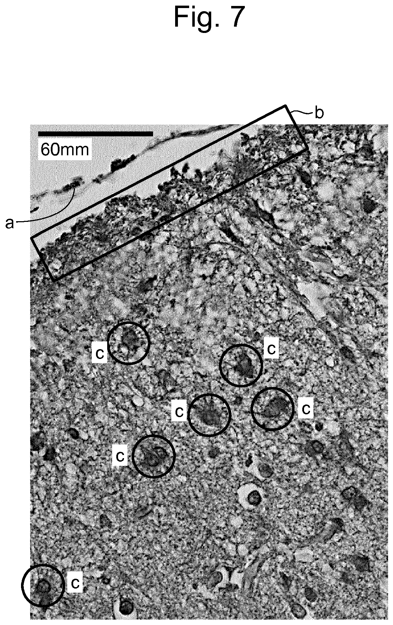

Figure 7 shows results of immunohistochemistry of the AD brain (cerebral cortex) using an anti-Tf antibody. In the drawing, "a" indicates the pia mater. "b" indicates positive fiber bundles in the box. "c" indicates astrocyte-like cells in the circled regions. In the AD brain, astrocyte-like cell bodies are stained. Positive fibers were observed in the neuropils of the first cortical layer (molecular layer) and arranged in bundles, particularly, beneath the pia mater.

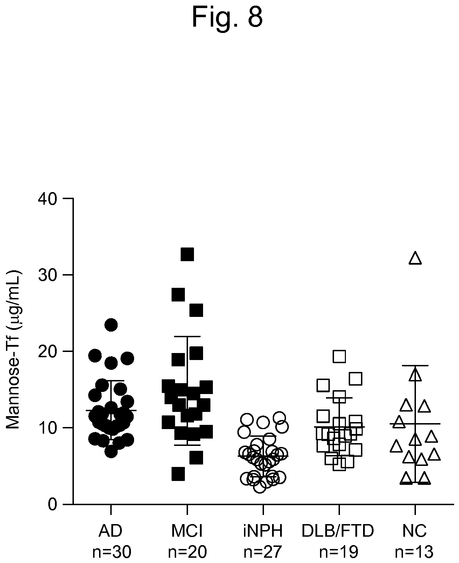

Figure 8 shows results of quantifying terminal Man-Tf in cerebrospinal fluid by BC2L-A lectin/antibody sandwich ELISA for 5 groups: an AD group, a MCI group, an iNPH group, a DLB/FTD group, and a NC group.

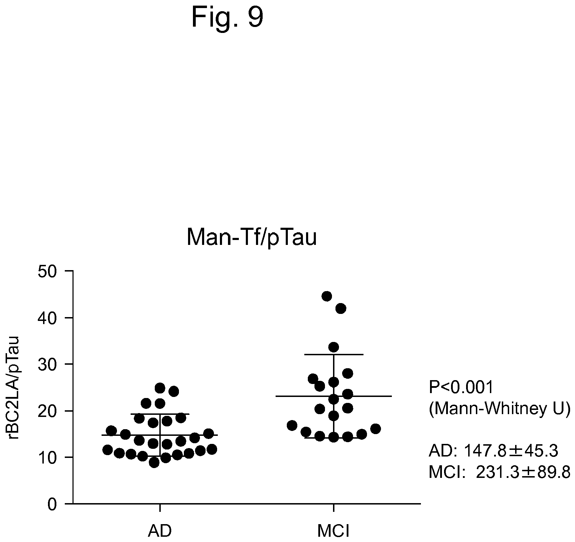

Figure 9 is a diagram showing the ratio of a measurement value of Man-Tf to a measurement value of phosphorylated tau protein (p-Tau) which is a known diagnostic marker for AD. Phosphorylated-Tau and Man-Tf, which is quantified by ELISA using BC2L-A lectin/antibody, were measured with the same cerebrospinal fluid sample.

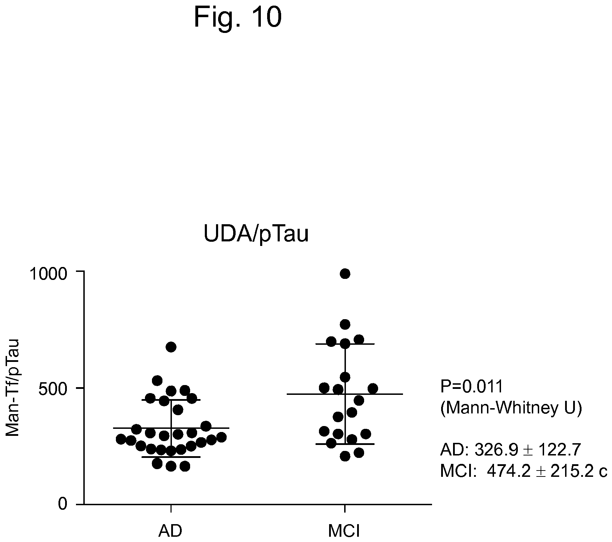

Figure 10 is a diagram showing the ratio of a measurement value of Man-Tf to a measurement value of phosphorylated tau protein (p-Tau) which is a known diagnostic marker for AD (Man-Tf/p-Tau) by ELISA using UDA lectin/antibody with the same cerebrospinal fluid sample.

### Description of Embodiments

### 1. Diagnostic marker for Alzheimer's disease

### 1-1. Summary

The first aspect of the present invention is a diagnostic marker for Alzheimer's disease (AD). The diagnostic marker of the present invention consists of a glycoprotein present in a body fluid, or a peptide fragment thereof and enables the diagnosis of the contraction or potential contraction of AD or the determination of a pathological condition of AD in a test subject.

### 1-2. Definition

The terms used in the present specification will be defined below.

In the present specification, the "Alzheimer's disease (AD)" is, as mentioned above, an irreversible progressive central nervous system disease that manifests symptoms such as dementia, behavioral disorder, or personality change. In the present specification, Alzheimer's disease transition-type mild cognitive impairment (AD transition-type MCI) is included in AD, as mentioned later.

In the present specification, the "mild cognitive impairment (MCI)" refers to a condition that causes dysfunction in one of the cognitive functions (memory, decision making, reasoning, action execution, etc.) and as a result, manifests slight reduction in cognitive function, which, however, does not interfere with daily life. In the present specification, MCI is included in central nervous system diseases. As mentioned above, some MCI patients have AD transition-type MCI which, through progression, transitions to AD in the future. Because of the eventual transition of the AD transition-type MCI to AD, the AD transition-type MCI is encompassed by the category of AD, not by the category of MCI, in the present specification.

In the present specification, the "diagnosis" refers to the identification of the contraction or potential contraction of a disease, or the judgement of a pathological condition of a disease. Typically, the diagnostic action is exclusive operation of doctors, veterinarians, or dentists. In the present specification, the diagnosis includes auxiliary action that assists diagnosis by doctors, etc. without the mediation of action by the doctors, etc.

In the present specification, the "identification of the contraction" refers to judgements of whether or not to have a particular disease. The identification of the contraction also includes the discrimination of the particular disease from other diseases having similar lesion or pathology.

In the present specification, the "potential contraction" refers to the probability of the contraction of a particular disease in the future. In the present specification, the potential contraction also includes the potential transition of MCI to AD when the particular disease is AD.

In the present specification, the "pathological condition of a disease" refers to the state of the particular disease, and the degree or stage thereof. Examples of the degree of the state include mild case (mild degree), moderate case (moderate degree), and severe case (severe degree). Examples of the stage include early stage, intermediate stage, and late stage. In the present specification, the "pathological condition" includes not only the state of the disease at a particular time point in a patient but also change in the state of the disease from a certain time point to another time point. The pathological condition may also include, for example, the progression status of the disease in which the state of the disease is worsened as the time passes, or the degree thereof, or on the contrary, the curing status of the disease in which the state of the disease is ameliorated as the time passes, or the degree thereof. Thus, in the present specification, the "determination of a pathological condition" includes the determination of the state or degree of a disease such as dementia at a particular time point, and the determination of change (progression or amelioration) in the state of the disease from a certain time point to another time point.

In the present specification, the "body fluid" refers to a liquid sample collected from a test subject or a control. Examples thereof include cerebrospinal fluid, blood (including serum, plasma and interstitial fluid), urine, lymph fluid, digestive juice, ascitic fluid, pleural effusion, nerve root fluid, and extracts of each tissue or cells. The body fluid is preferably cerebrospinal fluid or blood, more preferably cerebrospinal fluid.

In the present specification, the "cerebrospinal fluid (CSF) refers to a transparent colorless extracellular fluid that is present only around the central nerve system (CNS) of the brain and the spinal cord. The cerebrospinal fluid is separated from other organs by the dura mater and from blood by the blood-brain barrier or the blood-cerebrospinal fluid barrier. Recent studies revealed that a large part of the cerebrospinal fluid is effused from the parenchyma of the brain; and that there is virtually no barrier between brain cells and the cerebrospinal fluid (Wang C, et al., 2012, Cerebrospinal Fluid: Physiology, biomarker and methodology. In: V. S, Dolezal, T., editor. Cerebrospinal Fluid: Functions, Composition and Disorders. New York: Nova Science Publishers; pp. 1-37). In this cerebrospinal fluid, many proteins derived from the central nerve system are present, and their expression is known to increase or decrease in association with central nervous system diseases.

In the present specification, the "glycoprotein" refers to a protein with one or more glycan(s) attached thereto. The glycan in the glycoprotein is attached as one of the posttranslational modifications and is reportedly present in 50% or more of *in vivo* proteins. The glycan plays an important role in conferring various functions to the protein in such a way that the glycan is involved in protein stabilization, protection, biological activity, antigen-antibody reaction, viral infection and pharmacokinetics, etc.

In the present specification, the "peptide fragment (containing the glycan)" is a peptide that consists of a portion of the glycoprotein and contains an amino acid residue with the glycan attached thereto. In the present invention, the peptide fragment (containing the glycan) particularly refers to a peptide that consists of a portion of terminal Man-Tf which is the third Tf glycan isoform described in "1-3. Configuration" mentioned later, and contains an asparagine residue at position 432 to which a glycan terminating in non-reducing end mannose is attached. The number of amino acids of the peptide fragment is not particularly limited. Because the peptide fragment is a portion of the glycoprotein, the lower limit can be a length capable of including an epitope, for example, 8 amino acids or longer, preferably 10 or 15 amino acids or longer, more preferably 20 or 30 amino acids or longer. The upper limit can have amino acids fewer than those of the full length glycoprotein.

In the present specification, the "glycan terminating in non-reducing end mannose (glycan terminating in non-reducing end Man)" refers to a glycan having at least one mannose at a non-reducing end. The number of the non-reducing end Man is not limited. A single Man may be present at a non-reducing end of a linear glycan, or single or multiple Man may be present at a plurality of non-reducing ends formed by branching. In the present specification, the term "multiple (a plurality of)" refers to 2 to 10, 2 to 9, 2 to 8, 2 to 7, 2 to 6, 2 to 5, 2 to 4, or 2 or 3. It is preferred that all of non-reducing end sugars should be mannose. A high-Man (high-mannose) glycan or a hybrid glycan having mannose at at least one non-reducing end corresponds to a glycan terminating in non-reducing end Man.

In the present specification, the "non-reducing end (sugar)" of a glycan refers to a terminal sugar that does not have a free hydroxyl group and hence exhibits no reducing character, in the glycan of the glycoprotein. Examples thereof include non-reducing end mannose (non-reducing end Man), non-reducing end GlcNAc, and non-reducing end sialic acid (non-reducing end Sia). On the other hand, the "reducing end (sugar)" of a glycan is a terminal sugar that has a free hydroxyl group and exhibits a reducing character. The reducing sugar assumes a hemiacetal or ketal structure. Typically, the reducing end sugar is bound with a protein.

In the present specification, the "test subject" refers to a subject to be applied with each aspect of the present invention. The test subject is an individual as a rule, but also includes tissues or cells. Specific examples of the individual include individuals of mammals, preferably humans, dogs, cats, and horses. A human is preferred. The individual is unlimited to be living or dead, and a living organism is preferred. Also, the individual may be any of a disease-contracted subject having some disease, an individual with the potential contraction of a disease, and a normal subject.

In the present specification, the "disease-contracted subject" refers to an individual having a particular disease. In the present specification, the disease-contracted subject is typically, for example, an AD-contracted subject or a MCI-contracted subject (particularly, an AD transition-type MCI-contracted subject). Also, the "individual with the potential contraction of a disease" is, for example, an individual having the potential to transition from MCI to AD.

In the present specification, the "normal subject" refers to an individual having a normal condition. The "normal condition" means a state without the contraction of at least central nervous system diseases, for example, AD and MCI, preferably a healthy state without any disease or disorder. Thus, in the present specification, the "disease control" described in Examples mentioned later corresponds to one form of the normal subject. In the present specification, the normal subject is mainly used as a control serving as a reference for comparison with the test subject. Thus, in the present specification, the test subject and the normal subject are at least of the same species. It is more preferred that conditions such as subspecies (including race), sex, age (including age in month and age in week), height, and body weight should be the same. In the present specification, a population consisting of a plurality of normal subjects is referred to as a "normal group".

### 1-3. Configuration

The "diagnostic marker for Alzheimer's disease (diagnostic marker for AD)" of the present invention is a biomarker that is constituted by a glycoprotein or a glycan-containing peptide fragment and permits diagnosis of the contraction of AD or AD transition-type MCI or the potential contraction of AD, i.e., the potential to transition from MCI to AD, in a test subject.

In the diagnostic marker for AD of the present invention, the protein constituting the glycoprotein is transferrin (Tf) protein. The Tf protein is a carrier protein having a molecular weight of approximately 80 KDa that reversibly binds to two iron (Fe) ions and is responsible for the *in vivo* transport thereof. Specific examples of the transferrin include human Tf protein which is constituted by 698 amino acid residues and consists of the amino acid sequence represented by SEQ ID NO: 1. The Tf protein typically exerts its functions as a glycoprotein. In the present specification, such Tf protein with a glycan attached thereto is also referred to as a "Tf glycoprotein".

N-linked glycans attached to the side chain of an asparagine (Asn: N) residue in the amino acid sequences of proteins, and O-linked side chains attached to the side chain of a serine (Ser: S) residue or a threonine (Thr: T) residue therein are known as glycans. The glycan of the Tf glycoprotein constituting the diagnostic marker for AD of the present invention is N-linked glycans attached to two asparagine residues (N432 and N630), as mentioned above.

The Tf glycoprotein includes three isoforms (herein referred to as "Tf glycan isoforms") having a common protein moiety and differing only in the structures of the N-linked glycans attached thereto. The diagnostic marker for AD of the present invention is constituted by terminal Man-Tf which is one of the isoforms and corresponds to the third Tf glycan isoform mentioned later. Hereinafter, the three Tf glycan isoforms of the Tf glycoprotein will be described.

The first Tf glycan isoform corresponds to sialylated Tf mentioned above and is a Tf glycoprotein having a structure where two glycans terminating in non-reducing end sialic acid are attached thereto, as shown in Figure 1A. The "glycan terminating in non-reducing end sialic acid" refers to a glycan having α2,6-sialic acid at a non-reducing end. In the present specification, the Tf glycan isoform to which a glycan terminating in non-reducing end sialic acid is attached is often referred to as "terminal Sia-Tf (glycoprotein)". The terminal Sia-Tf is present in both serum and cerebrospinal fluid, as shown in the band "a" of Figure ID. The terminal Sia-Tf is also called "serum Tf (glycoprotein)" because this isoform is abundant, particularly, in serum. The terminal Sia-Tf is considered as a Tf glycoprotein that is biosynthesized in the liver because of its similarity to other glycoproteins in the glycan structure.

The second Tf glycan isoform corresponds to non-sialylated Tf subgroup 1 mentioned above and is a Tf glycoprotein to which two glycans terminating in non-reducing end GlcNAc are attached, as shown in Figure 1B. The "glycan terminating in non-reducing end GlcNAc" refers to a glycan having N-acetylglucosamine (GlcNAc) at a non-reducing end. In the present specification, the Tf glycan isoform to which a glycan terminating in non-reducing end GlcNAc is attached is often referred to as "terminal GlcNAc-Tf (glycoprotein)". The terminal GlcNAc-Tf is considered as a brain-type glycoprotein that is produced by cells of the central nerve system such as the brain and/or the spinal cord and found mainly in cerebrospinal fluid.

The third Tf glycan isoform corresponds to non-sialylated Tf subgroup 2 mentioned above and is a Tf glycoprotein in which a glycan terminating in non-reducing end Man is attached as one of the two glycans while a glycan terminating in non-reducing end GlcNAc is attached as the other glycan, as shown in Figure 1C. More specifically, as for the human Tf protein, a Tf glycoprotein corresponds thereto in which a glycan terminating in non-reducing end Man is attached to N432 while a glycan terminating in non-reducing end GlcNAc is attached to N630. In the present specification, the Tf glycan isoform having the glycan structure mentioned above is often referred to as "terminal Man-Tf (glycoprotein)". The terminal Man-Tf and the terminal GlcNAc-Tf are closely similar in mobility and are therefore detected as an apparently single glycan isoform in Western blot since their bands overlap as shown in the band "b" of Figure ID. Hence, the terminal GlcNAc-Tf has been known so far as a cerebrospinal fluid-specific Tf glycoprotein, whereas the presence of the terminal Man-Tf has not been known. The terminal Man-Tf is also considered as a brain-type glycoprotein, as is the case with the terminal GlcNAc-Tf.

As mentioned above, the second and third Tf glycan isoforms are brain-type glycoproteins and are therefore present mainly in cerebrospinal fluid. However, some components in cerebrospinal fluid (cerebrospinal fluid components) are known to be also present in head and neck lymph ducts, the spinal nerve root, etc. and also leaked, albeit in a very small amount, into serum. For example, prostaglandin D2 synthase, a brain-type glycoprotein unique to cerebrospinal fluid, is detectable with its concentration on the order of 1% in blood. Thus, the second and third Tf glycan isoforms may also be present in a body fluid other than cerebrospinal fluid.

Each Tf glycan isoform mentioned above is specific for cell types, and its quantitative change reflects the morbidity of the cell types of its origin. Hence, the Tf glycan isoform can serve as a useful diagnostic marker for a disease.

For example, the terminal GlcNAc-Tf is known to serve as an index marker for iNPH (JP Patent Publication (Kokai) No. 2010-121980 A (2010)).

On the other hand, the terminal Man-Tf is a Tf glycan isoform constituting the diagnostic marker for AD of the present invention, though this isoform does not serve as an index marker for iNPH. The amount of the terminal Man-Tf in a body fluid (mainly in cerebrospinal fluid) is significantly increased in an AD transition-type MCI-contracted subject and an AD-contracted subject compared with a normal subject or a normal group. Such increase is not observed in other central nervous system diseases. Thus, the terminal Man-Tf can also serve as a useful marker for discriminating AD transition-type MCI from non-AD transition-type MCI, and for discriminating AD from other central nervous system diseases similar thereto in lesion or pathology. For example, when the amount of the terminal Man-Tf is significantly larger in the body fluid (mainly in cerebrospinal fluid) of a patient diagnosed as having MCI by early examination based on interview or the like, as compared with a normal subject or a normal group, the MCI is AD transition-type MCI and can be determined to be highly likely to transition to AD in the future. On the other hand, when there is no significant difference in the amount of the terminal Man-Tf, the MCI can be determined as non-AD transition-type MCI.

For example, the symptoms of early AD are difficult to distinguish from the early symptoms of dementia with Lewy bodies (herein often referred to as "DLB") or frontotemporal dementia (herein often referred to as "FTD"). Thus, it is not easy to differentiate therebetween from symptomatological difference. Particularly, for FTD, it has been reported that an increased level of phosphorylated tau protein in a body fluid (mainly in cerebrospinal fluid) is seen, as in AD, and an existing, reportedly most effective diagnostic marker for AD cannot be utilized. However, DLB or FTD exhibits no significant difference in the amount of the terminal Man-Tf in a body fluid (mainly in cerebrospinal fluid), as compared with a normal subject or a normal group. Thus, AD can be easily differentiated from DLB or FTD by measuring the amount of the terminal Man-Tf in a body fluid (mainly in cerebrospinal fluid).

For example, late-stage AD and iNPH are very similar in that both of them show marked morphological change of ventricular enlargement, and dementia. Thus, it is not easy to differentiate therebetween. iNPH is caused by an excess of cerebrospinal fluid and is therefore healable completely by removing the excessive cerebrospinal fluid via simple minor operation (e.g., shunt operation of bypassing the cerebral ventricle to the peritoneal cavity with a tube). Namely, since iNPH is "curable" dementia, its precise diagnosis is demanded. Nonetheless, only 1200 patients (0.4% of the predicted number of patients) with this disease undergo radical surgery yearly in Japan, though the number of iNPH patients is estimated at 310,000 people. This suggests that many iNPH patients are misdiagnosed as AD and left untreated without receiving benefits from treatment. In recent years, many cases of comorbidity or coincidence of iNPH and AD have been reported. In general, the pathology of AD starts with the aggregation of Aβ, and the aggregates of Aβ become amyloid plaques observed in early-stage AD. However, normally, the aggregation of Aβ is suppressed because a biological mechanism works to wash out Aβ by the circulation of cerebrospinal fluid. Hence, in some iNPH cases, the pathology of AD is considered to easily progress as the wash-out of Aβ is reduced due to failure of the circulation of cerebrospinal fluid. In fact, it has been reported that when AD marker-positive cases (coincident cases) and -negative cases of iNPH patients are compared, recovery of memory, delayed recall, or the like is not favorable in the former (Kazui et al, 2016, J Neurol Sci, 369: 236-41. doi: 10.1016/j.jns.2016.08.040. Epub 2016 Aug 19). Thus, it is important for the prediction of a therapeutic effect to measure respective early diagnostic markers for iNPH and AD and differentiate between coincident and non-coincident cases. In this context, the terminal Man-Tf in a body fluid (mainly in cerebrospinal fluid) serves as a diagnostic marker for AD. On the other hand, the terminal GlcNAc-Tf, which is also one of the Tf glycan isoforms in the body fluid (mainly in cerebrospinal fluid), serves as a diagnostic marker for iNPH as mentioned above. Hence, in iNPH, no significant difference is observed in the amount of the terminal Man-Tf in a body fluid (mainly in cerebrospinal fluid) as compared with a normal subject or a normal group, whereas the amount of the terminal GlcNAc-Tf in the body fluid (mainly in cerebrospinal fluid) is significantly decreased. On the other hand, in AD or AD transition-type MCI, no significant difference is observed in the amount of the terminal GlcNAc-Tf in the body fluid (mainly in cerebrospinal fluid) as compared with a normal subject or a normal group, whereas the amount of the terminal Man-Tf therein is increased. Thus, the two diseases can be discriminated from each other easily and highly accurately by measuring their respective diagnostic markers present in a body fluid (mainly in cerebrospinal fluid).

In the present specification, the term "significant" refers to being statistically significant. The term "statistically significant" means that when the difference between a measurement value of a test subject and a control value is statistically processed, there is a significant difference therebetween. In the present invention, the term refers to a significant difference between measurement values of the amount of the terminal Man-Tf in a test subject and a normal group when statistically processed. Examples thereof include the case where the significance level of the obtained value is small, specifically, smaller than 5% (p < 0.05), smaller than 1% (p < 0.01), or smaller than 0.1% (p < 0.001). In this context, the term "p (value)" refers to the probability that a hypothesis is accidentally true in a hypothesized distribution of statistics in a statistical test. Thus, a smaller value of "p" means that the hypothesis is closer to truth. A test method known in the art, capable of determining the presence or absence of significance can be used as appropriate as a test method for the statistical processing, without particular limitations. For example, Student's t test, analysis of covariance, or the like can be used.

The amount of the terminal Man-Tf is increased in correlation with the degree of progression of the disease. Namely, a larger amount of the terminal Man-Tf in a body fluid (mainly in cerebrospinal fluid) as compared with a normal subject or a normal group means that the symptoms are more advanced and more severe. Conversely, when the disease has been cured, the amount of the terminal Man-Tf may decrease in correlation with the degree of recovery and may go back to that in a normal subject or a normal group. Hence, the terminal Man-Tf can serve as a diagnostic marker that reflects a pathological condition of AD.

### 2. Diagnostic kit for Alzheimer's disease

### 2-1. Summary

The second aspect of the present invention is a diagnostic kit for Alzheimer's disease (diagnostic kit for AD). The kit of the present invention can detect the diagnostic marker for AD described in the first aspect which may be contained in a sample and thereby, can identify the contraction or potential contraction of AD and/or determine a pathological condition of AD in a test subject who is a sample provider.

### 2-2. Configuration

The diagnostic kit for AD of the present invention includes a mannose binding molecule and a transferrin binding molecule as essential constituents. The diagnostic kit for AD of the present invention can also include other constituents as optional constituents.

### 2-2-1. Essential constituent

### (1) Mannose binding molecule

In the present specification, the "mannose binding molecule (Man binding molecule)" refers to a molecule recognizing and binding to mannose constituting a glycan of a glycoprotein. It is preferred that the recognition of and binding to the target molecule should be highly specific. The Man binding molecule may be any of a peptide, a nucleic acid, a low-molecular weight compound, and a combination thereof. In the present specification, the "low-molecular weight compound" refers to an organic compound or an inorganic compound having a molecular weight of 500 or smaller except for peptides and nucleic acids. The glycan mannose recognized by the Man binding molecule may be any of reducing end mannose, non-reducing end mannose, and mannose internal to the glycan. The mannose is preferably terminal mannose, more preferably non-reducing end mannose.

Specific examples of the Man binding molecule include mannose binding proteins and mannose binding nucleic acids. Hereinafter, each of these Man binding molecules will be described.

### (A) Mannose binding protein

Examples of the "mannose binding protein (Man binding protein)" include mannose binding lectins, and anti-mannose antibodies and active fragments thereof.

### (i) Mannose binding lectin

The "mannose binding lectin (Man binding lectin)" is a lectin binding to a glycan containing mannose, preferably a glycan containing terminal mannose, more preferably a glycan containing non-reducing end mannose. Examples thereof include 45 mannose binding lectins described in http://jcggdb.jp/rcmg/glycodb/LectinSearch. The apparent glycan binding specificity of a lectin may differ when the target molecule is a glycoprotein or a glycan alone. For example, *Urtica dioica*-derived UDA lectin is classified into a GlcNAc binding lectin when the target molecule is a glycan. However, when the target molecule is a glycoprotein, this UDA lectin specifically binds to non-reducing end Man of terminal Man-Tf, whereas it does not bind to terminal GlcNAc-Tf, as shown in Figure 2. Thus, it is preferred that the binding of the lectin to mannose should be studied using the target molecule, i.e. terminal Man-Tf, rather than the glycan alone. Namely, the Man binding lectin constituting the kit of the present invention is a lectin binding to glycan mannose of the terminal Man-Tf, preferably terminal mannose of the terminal Man-Tf, more preferably non-reducing end Man of the terminal Man-Tf. A commercially available lectin may be used as the Man binding lectin. For example, biotinylated UDA lectin (Cat No BA-8005-1; EY Laboratories, Inc.) or bacterium-derived BC2L-A lectin (Burkholderia cenocepacia lectin-A; Wako Pure Chemical Industries, Ltd.) can be used.

### (ii) Anti-mannose antibody or active fragment thereof

The "anti-mannose antibody (anti-Man antibody)" is an antibody recognizing and binding to a glycan containing mannose, preferably a glycan containing terminal mannose, more preferably a glycan containing non-reducing end Man.

The "active fragment thereof' refers to a partial fragment of the antibody that retains antigen binding activity or immune response activity.

The anti-Man antibody may be any of a polyclonal antibody, a monoclonal antibody and a recombinant antibody. A monoclonal antibody or a recombinant antibody is preferred for enabling more specific detection. The globulin type of the antibody is not particularly limited and may be any of IgG, IgM, IgA, IgE, IgD, and IgY. IgG and IgM are preferred. The organism species from which the antibody of this aspect is derived is not particularly limited. The antibody can be derived from any animal including mammals and bird. Examples thereof include mice, rats, guinea pigs, rabbits, goats, donkeys, sheep, camels, horses, chickens, and humans.

In the present specification, the "recombinant antibody" refers to, for example, a chimeric antibody, a humanized antibody and a synthetic antibody.

The "chimeric antibody" is an antibody in which light chain and heavy chain constant regions (C regions) in a certain antibody are replaced with light chain and heavy chain C regions of a different antibody. For example, an antibody corresponds thereto in which light chain and heavy chain C regions of a mouse anti-human monoclonal antibody are replaced with C regions of an appropriate human antibody. In this case, variable regions (V regions) containing CDRs are derived from the mouse antibody, and the C regions are derived from the human antibody.

The "humanized antibody" is also called reshaped human antibody and is a mosaic antibody in which CDRs of a nonhuman mammal-derived antibody against a target antigen are replaced with CDRs of a human antibody. For example, an antibody corresponds thereto which is obtained by replacing a DNA sequence encoding each CDR region (CDR1 to CDR3) of a mouse-anti-human non-reducing end Man antibody with a DNA sequence encoding each corresponding CDR derived from an appropriate human antibody to prepare a recombinant antibody gene, which is then expressed.

The "synthetic antibody" refers to an antibody synthesized by use of a chemical method or a recombinant DNA method. For example, a monomeric polypeptide molecule in which one or more VLs and one or more VHs of a particular antibody are artificially linked via a linker peptide or the like having an appropriate length and sequence, or a multimeric polypeptide thereof corresponds thereto. Specific examples of such a polypeptide include single chain Fv (scFv: single chain fragment of variable region) (see Pierce Catalog and Handbook, 1994-1995, Pierce Chemical Co., Rockford, IL), diabody, triabody and tetrabody. In an immunoglobulin molecule, VL and VH are typically positioned on separate polypeptide chains (light chain and heavy chain). The single chain Fv is a synthetic antibody fragment having a structure where the V regions on these two polypeptide chains are linked via a flexible linker having a sufficient length so as to be included in a single polypeptide chain. The two V regions within the single chain Fv can be self-assembled with each other to form a single functional antigen binding site. The single chain Fv can be obtained by integrating recombinant DNA encoding it into phage genome by use of a technique known in the art, followed by expression. The diabody is a molecule having a structure based on the dimer structure of single chain Fv (Holliger et al., 1993, Proc. Natl. Acad. Sci. USA 90: 6444-6448). The triabody and the tetrabody have trimer and tetramer structures, respectively, based on the single chain Fv structure, as in the diabody. These molecules are trivalent and tetravalent antibody fragments, respectively, and may be multispecific antibodies.

It is preferred that the anti-Man antibody should have high affinity of 10⁻⁸ M or less, preferably 10⁻⁹ M or less, more preferably 10⁻¹⁰ M or less, in terms of a dissociation constant for the terminal Man-Tf. The dissociation constant can be measured by use of a technique known in the art. The dissociation constant may be measured using, for example, rate evaluation kit software from BIAcore system (GE Healthcare Japan Corp.).

The polyclonal antibody of this aspect for use in this step can be obtained by immunizing an appropriate animal with a glycan having non-reducing end Man, or a glycoprotein fragment consisting of several amino acids including the asparagine residue to which the glycan is attached, as an antigen, followed by collection from the immunized animal by a method known in the art. The monoclonal antibody can also be obtained by a method known in the art which is a technique routinely used in the art. For example, a mouse is immunized with the antigen, and antibody-producing cells are then collected from the immunized mouse. The antibody-producing cells are fused with a myeloma cell line. Hybridoma cells are thereby produced. Finally, a hybridoma producing the monoclonal antibody binding to the target molecule, i.e. terminal Man-Tf, can be identified.

The "antibody fragment" is a peptide fragment having the antigen binding activity of the antibody mentioned above. Examples thereof include Fab, F(ab')₂, and Fv.

The antibody or the antibody fragment thereof for use in this step may be modified. In this context, the "modification" includes labeling necessary for antibody detection, or functional modification necessary for antigen-specific binding activation. Examples of the labeling include labeling with fluorescent materials mentioned above, fluorescent proteins (e.g., PE, APC, and GFP), enzymes (e.g., horseradish peroxidase, alkaline phosphatase, and glucose oxidase), and biotin or (strept)avidin. Examples of the modification include antibody glycosylation that is performed in order to adjust the affinity for the glycan having non-reducing end Man in the target molecule, i.e. terminal Man-Tf. Specific examples thereof include engineering by which a substitution is introduced to amino acid residues constituting a glycosylation site in FR (framework region) of an antibody to remove the glycosylation site, thereby deleting glycosylation at this site.

### (B) Mannose binding nucleic acid

Examples of the "mannose binding nucleic acid (Man binding nucleic acid)" include nucleic acid aptamers against mannose.

The "nucleic acid aptamer" refers to an aptamer constituted by a nucleic acid, which is a ligand molecule having the ability to bind strongly and specifically to a target substance through a conformation formed on the basis of the secondary structure and tertiary structure of a single-stranded nucleic acid molecule via a hydrogen bond or the like. Specifically, the nucleic acid aptamer is a nucleic acid aptamer recognizing and binding to mannose, preferably non-reducing end mannose, and glycan structure around it.

The nucleic acid aptamer used in the present specification can be prepared by a method known in the art. Examples thereof include an *in vitro* selection method using SELEX (systematic evolution of ligands by exponential enrichment). The SELEX method, for example, in the case of separating an RNA aptamer, involves "selecting an RNA molecule bound to a target molecule from an RNA pool constituted by many RNA molecules each having a random sequence region and primer binding regions at both ends thereof, amplifying the recovered RNA molecule through RT-PCR reaction, then performing transcription with the obtained cDNA molecule as a template to obtain an amplification product of the selected RNA molecule, and using the amplification product as an RNA pool for the subsequent round". This series of cycles is repeated in several to several tens of rounds to select an RNA molecule having the ability to bind more strongly to the target molecule. On the other hand, in the case of separating a DNA aptamer by SELEX, it is not necessary to mediate RT-PCR reaction for amplifying the recovered DNA molecule, though the basic procedures are the same as above. The nucleotide sequence lengths of the random sequence region and the primer binding regions are not particularly limited. In general, the random sequence region is preferably in the range of 20 to 80 bases, and each of the primer binding regions is preferably in the range of 15 to 40 bases. Specificity for the target molecule can be enhanced by premixing a molecule similar to the target molecule with RNA pools or RNA pools and using a pool consisting of RNA molecules or DNA molecules that have not bound to the molecule similar to the target molecule. The SELEX method is a method known in the art, and its specific procedures can be performed in accordance with, for example, Pan et al. (Proc. Natl. Acad. Sci. U.S.A., 1995, 92: 11509-11513).

In the present invention, the method described above is carried out with glycan mannose as the target molecule. The nucleic acid molecule thus finally obtained can be used as the nucleic acid aptamer against mannose.

In general, RNA aptamers and DNA aptamers are known as nucleic acid aptamers. In the present specification, the nucleic acid constituting the nucleic acid aptamer is not particularly limited. The nucleic acid aptamer includes, for example, a DNA aptamer, an RNA aptamer, and an aptamer constituted by combinations of DNA and RNA. In general, an RNA aptamer is frequently used. A DNA aptamer is superior in stability, production cost for chemical synthesis, and the number of steps in aptamer production.

The nucleic acid aptamer for use in this step may be labeled with a fluorescent substance (e.g., FITC, Texas, Cy3, Cy5, Cy7, Cyanine3, Cyanine5, Cyanine7, FAM, HEX, VIC, fluorescamine and derivatives thereof, and rhodamine and derivatives thereof), a radioisotope (e.g., ³²P, ³³P, and ³⁵S), or a labeling substance such as biotin or (strept)avidin, without inhibiting the ability to bind to the target molecule.

### (2) Tf binding molecule

In the present specification, the "Tf binding molecule" refers to a substance recognizing and binding to the Tf protein constituting the protein moiety of the target molecule, i.e. terminal Man-Tf. It is preferred that the recognition of and binding to the target molecule should be highly specific. Examples of such a substance include peptides, nucleic acids, low-molecular weight compounds, and combinations thereof.

Specific examples of the Tf binding molecule include Tf binding proteins and Tf binding nucleic acids. Hereinafter, each of these Tf binding molecules will be described.

### (A) Tf binding protein

Examples of the "Tf binding protein" include anti-Tf antibodies and active fragments thereof.

### (i) Anti-Tf antibody

The "anti-Tf antibody" is an antibody recognizing and binding to the Tf protein, preferably the Tf protein moiety of the terminal Man-Tf.

The configuration of the anti-Tf antibody of the present invention and the method for preparing the same are specifically described in the preceding section "(ii) Anti-mannose antibody or active fragment thereof' of "(1) Mannose binding molecule (A) Mannose binding protein". The present invention also conforms thereto, so that the description will be omitted here.

### (B) Tf binding nucleic acid

Examples of the "Tf binding nucleic acid" include nucleic acid aptamers against Tf protein.

The configuration of the nucleic acid aptamer and the method for preparing the same are specifically described in the preceding section "(1) Mannose binding molecule (B) Mannose binding nucleic acid". The present invention also conforms thereto, so that the description will be omitted here. In the present invention, the method described above is carried out with Tf protein as the target molecule. The nucleic acid molecule thus finally obtained can be used as the nucleic acid aptamer against Tf protein.

### 2-2-2. Optional constituent

The kit of the present embodiment may further include an albumin binding molecule as an optional constituent. Specific examples of the albumin binding molecule include Blue Sepharose, and anti-albumin antibodies and active fragments thereof.

The Man binding molecule, the Tf binding molecule and/or the albumin binding molecule described above may be immobilized on a carrier or labeled with a fluorescent dye or a luminescent material, etc., if necessary.

In addition to the constituents described above, the kit of the present embodiment can contain, for example, a column (spin column, etc.) to be packed with the Man binding molecule, the Tf binding molecule and/or the albumin binding molecule, a buffer for washing (PBS buffer, physiological saline, etc.), and/or an instruction stating a protocol or the like.

The kit of the present embodiment may further contain a substance binding to a particular diagnostic marker for a central nervous system disease. In this context, the "substance binding to a particular diagnostic marker for a central nervous system disease" refers to a substance that can specifically recognize and bind to a known index marker for a central nervous system disease. For example, an antibody against a particular terminal GlcNAc glycoprotein, or an active fragment thereof corresponds thereto. Specific examples thereof include antibodies against terminal GlcNAc-Tf serving as an index marker for iNPH (anti-terminal GlcNAc-Tf antibodies) and active fragments thereof.

### 3. Method for identifying contraction of Alzheimer's disease

### 3-1. Summary

The third aspect of the present invention is a method for identifying the contraction of AD. The method for identifying the contraction of AD according to the present invention can identify the contraction of AD in a test subject by measuring an amount of the diagnostic marker for AD of the first aspect present in a body fluid derived from the test subject, and comparing this measurement value with an amount of the diagnostic marker for AD present in body fluids derived from a control group.

### 3-2. Method

The method for identifying the contraction of AD according to the present invention comprises (1) a measurement step and (2) a comparison and identification step as essential steps. Hereinafter, each of the steps will be specifically described.

### (1) Measurement step

The "measurement step" is the step of measuring an amount of the AD marker described in the first aspect contained per predetermined amount of a body fluid, preferably cerebrospinal fluid, obtained from each of a test subject and a control group to obtain measurement values.

The "test subject" in this aspect refers to an animal individual that is subjected to the identification method of this aspect for the purpose of detecting AD. The test subject may be any of, for example, an individual whose contraction of a central nervous system disease is unknown, such as a medical examinee of checkup, and an individual confirmed to have some central nervous system disease, wherein the disease name is not identified. In the former case, the presence or absence of the contraction of AD in the test subject can be identified. In the latter case, the central nervous system disease in the test subject can be determined as AD or other central nervous system diseases.

The "control" in this aspect refers to an individual serving as a reference for comparison with the test subject in the identification method of this aspect. In an ordinary method for identifying the contraction of AD, a normal subject suffices as the control. In the identification method of this aspect, in the case of determining a pathological condition of AD, the control may be set to an AD transition-type MCI-contracted subject or an early AD-contracted subject in order to establish a high reference value for comparison. In the present specification, a population consisting of a plurality of controls is referred to as a "control group". In this context, the term "plurality" is preferably 3 or more. Examples of the control group include normal groups, AD transition-type MCI groups, and early AD groups. Each control constituting the control group is an individual having a similar condition as a rule.

The phrase "body fluid obtained from" refers to a body fluid collected from each of the test subject and the control group. Cerebrospinal fluid or blood is preferred. The method for collecting cerebrospinal fluid and blood can be any known method without particular limitations. For example, the cerebrospinal fluid can be collected by lumbar puncture. The lumbar puncture is a method that has relatively low invasiveness and is suitable for collecting cerebrospinal fluid, because this method is capable of controlling pain to a level equal to or less than that of blood collection by using a commercially available local anesthetic in advance, and can reduce adverse reactions by using an atraumatic needle. The blood can be collected according to a blood collection method known in the art. The body fluids of the test subject and the control are body fluids of the same type with each other as a rule, in such a way that one of the body fluids is cerebrospinal fluid if the other body fluid is also cerebrospinal fluid.

The "predetermined amount" refers to an amount predetermined depending on volume or weight. The predetermined amount is not particularly limited and needs to be an amount that permits measurement of the diagnostic marker for AD described in the first aspect contained at least in a body fluid, preferably cerebrospinal fluid, of an AD patient. The predetermined amount can be, for example, 5 µL to 1 mL of cerebrospinal fluid, or 5 µg to 200 µg in terms of the amount of a cerebrospinal fluid protein. The predetermined amount of the body fluid to be measured is the same between the test subject and the control.

In the present specification, the "measurement value" is a value that indicates the amount of the diagnostic marker for AD measured in this step. The measurement value may be an absolute value such as a volume or weight, or may be a relative value such as concentration, ionic strength, absorbance or fluorescence intensity.

In this step, the amount of the diagnostic marker for AD contained in the predetermined amount of each of a body fluid derived from a test subject (referred to as a "test body fluid"), preferably cerebrospinal fluid derived from a test subject (referred to as "test cerebrospinal fluid"), and a body fluid derived from a control group (referred to as a "control body fluid"), preferably cerebrospinal fluid derived from a control group (referred to as "control cerebrospinal fluid"), is measured using a Man binding molecule and a Tf binding molecule.

The configurations of the Man binding molecule and the Tf binding molecule are mentioned in detail in the second aspect, so that the specific description will be omitted here. One example of the Man binding molecule includes a Man binding lectin such as UDA lectin or BC2L-A lectin. One example of the Tf binding molecule includes an anti-Tf antibody.

The method for measuring the diagnostic marker for AD can be any glycoprotein quantification method known in the art which involves performing measurement using the Man binding molecule and the Tf binding molecule, without particular limitations. Examples thereof include immunological detection methods using an antibody, lectin detection methods using a lectin, mass spectrometry, and combined methods thereof.

Examples of the immunological detection methods include enzymatic immunoassay (including ELISA and EIA), fluorescent immunoassay, radioimmunoassay (RIA), luminescent immunoassay, surface plasmon resonance (SPR), quartz crystal microbalance (QCM), immunoturbidimetry, latex agglutination immunoassay, latex turbidimetry, hemagglutination reaction, particle agglutination reaction method, colloidal gold method, capillary electrophoresis, Western blot and immunohistochemistry (immunostaining method).

Examples of the lectin detection methods include lectin blotting.

Examples of the mass spectrometry include high-performance liquid chromatography-mass spectrometry (LC-MS), high-performance liquid chromatography-tandem mass spectrometry (LC-MS/MS), gas chromatography-mass spectrometry (GC-MS), gas chromatography-tandem mass spectrometry (GC-MS/MS), capillary electrophoresis-mass spectrometry (CE-MS) and ICP mass spectrometry (ICP-MS).

For example, sandwich ELISA using a lectin and an antibody and an automated method using principles similar thereto, preferably high-throughput lectin inhibition-automated latex agglutination, can be used in the combined method. The combined method for detection targeting both the glycan and the protein of the terminal Man-Tf constituting the diagnostic marker for AD can quantify the glycoprotein highly accurately and is therefore a preferred measurement method. Specific examples of the combined method include a method which involves applying a body fluid (e.g., cerebrospinal fluid) containing the terminal Man-Tf serving as the diagnostic marker for AD through an anti-Tf antibody-adsorbed carrier such as a plate to adsorb the terminal Man-Tf thereonto, followed by the detection of the terminal Man-Tf using the Man binding molecule, i.e. UDA lectin, as a probe. An alternative method involves applying a body fluid (e.g., cerebrospinal fluid) through a UDA lectin-adsorbed carrier such as a plate to adsorb the terminal Man-Tf thereonto, followed by the detection of the terminal Man-Tf using the anti-Tf antibody.

All of these analysis methods are techniques known in the art and can be performed in accordance with these methods. These analysis methods can be performed in accordance with methods described in, for example, Green, M.R. and Sambrook, J., 2012, Molecular Cloning: A Laboratory Manual Fourth Ed., Cold Spring Harbor Laboratory Press, Cold Spring Harbor, New York; Christopher J., et al., 2005, Chemical Review, 105: 1103-1169; Iijima Y. et al., 2008, The Plant Journal, 54, 949-962; Hirai M. et al., 2004, Proc Natl Acad Sci USA, 101(27) 10205-10210; Sato S, et al., 2004, The Plant Journal, 40 (1) 151-163; and Shimizu M. et al., 2005, Proteomics, 5, 3919-3931. Protein quantification kits are also commercially available from each manufacturer and may be used.

Unlike the measurement value of the test subject, the measurement value of the control group in the measurement step is not necessarily required to be measured in each case. For example, a previously measured measurement value of the control group can be reused as long as the predetermined amount of the body fluid (e.g., cerebrospinal fluid), the method for measuring the diagnostic marker for AD, and the measurement conditions are kept constant. Hence, the amount of the diagnostic marker for AD obtained from body fluids of controls are measured in advance under various conditions, and a database thereof is compiled. For comparison with the measurement value of the test subject, measurement values measured under the same conditions thereas may be selected and used according to the need.

### (2) Comparison and identification step

The "comparison and identification step" is the step of comparing the measurement values of the diagnostic marker for AD in the test subject and the control group, and identifying the contraction of AD or determining a pathological condition of AD in the test subject from the results.

In this step, the measurement values of the diagnostic marker for AD of the same type in the test subject and the control group obtained in the measurement step are compared. The measurement value of the control group can be an average measurement value of the individual controls constituting the control group. Also, a protein known in the art that does not quantitatively differ in cerebrospinal fluid may be used as an endogenous control for correcting the measurement values of the test subject group and the control group. Examples of such a protein for an endogenous control include albumin.

From the results of comparison, identification is performed as follows according to the control group.

In the case of using a normal group as the control group, the test subject is identified as having AD when the measurement value of the test subject is significantly higher than that of the control group. In this case, provided that the test subject has already been found to have any central nervous system disease, the test subject can be identified as having AD and having no other central nervous system diseases except for MCI related to AD.

In the case of using a MCI group as the control group, the potential that the test subject transitions from MCI to AD is identified as being high when the measurement value of the test subject is significantly higher than that of the control group.

In the case of using an early AD group as the control group, AD in the test subject is determined as advanced AD when the measurement value of the test subject is significantly higher than that of the control group. The pathological condition of AD in the test subject is determined to be keeping the status quo when there is no significant difference.

### 4. Method for identifying contraction of Alzheimer's disease transition-type mild cognitive impairment

### 4-1. Summary

The fourth aspect of the present invention is a method for identifying the contraction of AD transition-type MCI. The method for identifying AD transition-type MCI according to the present invention can identify the contraction of AD transition-type MCI in a test subject having MCI by measuring an amount of the diagnostic marker for AD of the first aspect present in a body fluid, preferably cerebrospinal fluid, derived from the test subject, and comparing this measurement value with an amount of the diagnostic marker for AD present in body fluids, preferably cerebrospinal fluid, derived from a normal group.

### 4-2. Method

The method for identifying the contraction of AD transition-type MCI according to the present invention also comprises a measurement step and a comparison and identification step as essential steps, as in the method for identifying the contraction of AD according to the third aspect. The configuration and procedures of each step are also the same as those of the method for identifying the contraction of AD according to the third aspect as a rule, so that only points different from the third aspect will be described here.

Examples of the different points of the method for identifying the contraction of AD transition-type MCI according to the present invention from the method for identifying the contraction of AD according to the third aspect include that the control is limited to a normal subject and that the test subject is a MCI-contracted subject having MCI.

As mentioned above, MCI includes the case where symptoms do not progress, and the case where symptoms progress to AD. The latter is AD transition-type MCI. It has been difficult to differentiate between common MCI and AD transition-type MCI by a conventional technique. Provided that an AD transition-type MCI patient among MCI patients can be identified, early treatment such as the administration of a therapeutic drug for AD can be started so that the transition to AD can be inhibited by circumventing massive neuronal death. Namely, provided that the contraction of AD transition-type MCI can be identified, the development of AD can be prevented.

In the case that the test subject is a MCI-contracted subject, the test subject is identified as having AD transition-type MCI in the comparison and identification step when the measurement value of the test subject is significantly higher than that of the normal group.

### Examples

### <Experimental Example 1: Binding specificity of UDA lectin>

### (Purpose)

The glycan binding specificity of UDA lectin used in the present Examples was tested.

### (Method)

Preparations of three Tf glycan isoforms were prepared. Terminal Sia-Tf having α2,6-sialic acid at non-reducing ends was obtained by purification from serum. This terminal Sia-Tf was digested with sialidase and galactosidase. This treatment yielded a Tf glycan isoform preparation having galactose and GlcNAc (terminal GlcNAc-Tf) at the non-reducing ends of the terminal Sia-Tf glycans. Subsequently, the terminal GlcNAc-Tf was further digested with hexosaminidase to obtain a Tf glycan isoform preparation having mannose (terminal Man-Tf). Here, the terminal Man-Tf is terminal Tri-Man-Tf having three glycans terminating in Man at non-reducing ends.

Electrophoresis was performed using these preparations, i.e., the terminal Sia-Tf, the terminal GlcNAc-Tf, and the terminal Man-Tf. The amount of each glycoprotein used in the electrophoresis was set to 80 ng for silver staining, 80 ng for immunoblot, and 160 ng for UDA lectin blot. A LaemmLi sample buffer was added to each glycoprotein, which was then heated and then subjected to SDS/PAGE using polyacrylamide gel having a gradient from 5 to 20%. The electrophoresis was carried out at a constant current of 20 mA for 75 minutes. The separated protein was stained using a commercially available silver staining kit (Cat No. 291-50301; Wako Pure Chemical Industries, Ltd.). Likewise, the separated protein was electrically transferred to nitrocellulose membranes at 350 mA for 45 minutes. One of the blots was blocked with 3% skimmed milk-PBST (phosphate-buffered saline-0.1% Tween) and then reacted with 0.5 µg/mL of an anti-Tf antibody (Cat No. A80-128A; Bethyl Laboratories, Inc.) at 24°C for 2 hours. For detection, the blot was reacted with 0.13 µg/mL of a horseradish peroxidase-labeled secondary antibody (anti-goat IgG antibody; Cat No. 705-035-147; Jackson ImmunoResearch Laboratories, Inc.). Then, color was developed using SuperSignal West Dura Extended Duration Substrate (Cat No. 34075; Pierce Chemical Co.), followed by image analysis using a chromatoscanner (AE-9300H Ez-Capture MG Quick Manual for ImageSaver 6; ATTO Corp.). The other blot was blocked with 1% BSA-PBS and then reacted with 1 µg/mL of biotinylated UDA (Cat No. BA-8005-1; EY Laboratories, Inc.) at 24°C for 90 minutes. Then, the blot was reacted with 1 mg/mL of HRP-labeled streptavidin (StAv-HRP; Cat No. 21126; Pierce Chemical Co.) at 24°C for 90 minutes. Then, color was developed using SuperSignal West Dura Extended Duration Substrate (Cat No. 34075; Pierce Chemical Co.), followed by detection using a chromatoscanner in the same way as in the immunoblot described above.

### (Results)

The results are shown in Figure 2. The three purified Tf glycan isoforms each exhibited a single major band by silver staining after electrophoresis (Figure 2A). Also, the three Tf glycan isoforms exhibited the same level of reactivity with the anti-Tf antibody (Figure 2B). Nonetheless, only the terminal Man-Tf gave signals in UDA lectin blot, whereas no signal was observed in the terminal GlcNAc-Tf and the terminal Sia-Tf (Figure 2C). This result indicated that the UDA lectin is a probe specific for the terminal Man-Tf.

### <Example 1: Detection of Tf in cerebrospinal fluid>

### (Purpose)

Tf in cerebrospinal fluid was actually detected using UDA lectin.

### (Method)

Three mL of cerebrospinal fluid was collected by lumbar puncture from each of 3 AD patients and 4 disease controls for controls after obtainment of informed consent. Subsequently, the collected cerebrospinal fluid samples were analyzed by UDA lectin blotting. The UDA lectin blot conformed to Experimental Example 1. The "disease control" refers to a non-central nervous system disease patient having no central nervous system disease. For example, a patient corresponds thereto who has undergone cerebrospinal fluid collection because of being suspected of having a neurological disease due to headache, neuralgia, vertigo, or the like, but has consequently been diagnosed with no neurological disease.

### (Results)

The results are shown in Figure 3. A band was detected around 74 kDa, which was consistent with the mobility of Tf, from all of the AD patients (AD) and the disease controls (Control). Quantification in a chromatoscanner showed that this signal intensity was increased by approximately 1.5 times in the cerebrospinal fluid of the three AD patients.

### <Example 2: Analysis by immunoprecipitation on terminal Man-Tf in cerebrospinal fluid>

### (Purpose)

Whether Tf detected around 74 kDa from cerebrospinal fluid using UDA lectin in Example 1 was derived from terminal Man-Tf was tested by immunoprecipitation.

### (Method)

The UDA lectin binds to every glycoprotein having non-reducing end mannose (terminal Man glycoprotein). Accordingly, whether or not there would exist a terminal Man glycoprotein, in cerebrospinal fluid, exhibiting the same mobility as that of a terminal Man-Tf (glycoprotein providing noise signals) was tested through the use of the phenomenon.

Immunobeads were prepared by binding an anti-Tf antibody to Protein G Sepharose 4 Fast Flow (Cat No. 17-0618-02; GE Healthcare Japan Corp.). The beads were incubated with cerebrospinal fluid at 4°C for 3 hours. After the incubation, the supernatant was collected as a non-binding fraction of the beads. Then, the beads were washed three times with TBST (Tris buffered saline-0.05% Tween). Then, a LaemmLi sample buffer was added to the beads, followed by the elution of a binding fraction. Each of unadjusted cerebrospinal fluid, the supernatant fraction, and the binding fraction was analyzed by electrophoresis (silver staining), immunoblot using an anti-Tf antibody, and lectin blot using UDA lectin in the same way as in Experimental Example 1 described above.

### (Results)

The results are shown in Figure 4.

In the silver staining shown in Figure 4A, two bands (a and b) were detected around 74 kDa in cerebrospinal fluid (lane 1). These two bands disappeared from the supernatant fraction of immunoprecipitation (lane 2) and were recovered in the binding fraction (lane 3), suggesting that both the bands were of Tf. Since this electrophoresis was performed in the presence of an added reducing agent, the two Tf bands in cerebrospinal fluid were close to each other.

In the immunoblot using an anti-Tf antibody, shown in Figure 4B, the bands "a" and "b" exhibited reactivity with the anti-Tf antibody (lane 3) and were thus actually confirmed to be of Tf.

In the lectin blot shown in Figure 4C, only the band "b" on the lower molecular weight side exhibited reactivity with the UDA lectin (lane 3). Nagae et al. (Glycobiology, 2014, 24, 693-702) discloses that a band on the lower molecular weight side is of a mixture of terminal Man-Tf and terminal GlcNAc-Tf (in the present specification, also referred to as "terminal Man-Tf/terminal GlcNAc-Tf"). This is consistent with the results described above, in light of the fact that the UDA lectin detected only the terminal Man-Tf. Thus, it was demonstrated that Tf detected around 74 KDa from cerebrospinal fluid using UDA lectin was terminal Man-Tf.

In the supernatant fraction, a weak band (c) was detected on a slightly lower molecular weight side than that of the terminal Man-Tf. This was considered to be a mannose-containing glycoprotein (GP-X) other than the terminal Man-Tf. The terminal Man-Tf was found to be quantifiable by lectin blot even in the presence of GP-X.

### <Example 3: Development of UDA lectin/antibody sandwich ELISA>

### (Purpose)

The lectin blot requires 2 days for the analysis of 20 samples. Accordingly, UDA lectin/antibody sandwich ELISA was developed as a quantification method having high measurement efficiency and high specificity for terminal Man-Tf.

### (Method)

2 µg/mL of an anti-Tf antibody (Cat No. A80-128A; Bethyl Laboratories, Inc.) or an anti-human transferrin monoclonal antibody (clone 7B2; Mikuri Immunological Lab. Co.) diluted with 0.05 M Carbonate-Bicarbonate pH 9.6 buffer was added to each well of a microtiter plate and incubated overnight at 4°C. The plate was washed once with TBS-0.05% Tween buffer and then blocked with 10% N101 (Cat No. S410-03012; NOF Corp.)-TBS for 1 hour or longer. Subsequently, cerebrospinal fluid was denatured in the presence of a surfactant. After washing the plate once with a buffer, the cerebrospinal fluid or 15 to 150 ng/mL of terminal Man-Tf diluted with a buffer was added to each well and incubated. The plate was washed with a buffer, and biotinylated UDA lectin was then added to each well and incubated. The plate was washed five times with a buffer, and horseradish peroxidase (HRP)-labeled streptavidin was then added to each well and incubated. The plate was washed five times with a buffer, and a chromogenic substrate TMB was then added to each well. Color was developed at room temperature, and the reaction was terminated with 1 M phosphoric acid, followed by the measurement of OD₄₅₀. A calibration curve was prepared using a terminal Tri-Man-Tf preparation, and the terminal Man-Tf in the cerebrospinal fluid was quantified.

### (Results)

The results are shown in Figure 5. The calibration curve obtained using a terminal Tri-Man-Tf preparation exhibited linearity in the range of 10 to 150 ng/mL. This preparation exhibited a value of 78 to 91% in spiking experiments for cerebrospinal fluid. The day-today reproducibility was within 10.5%. These results indicated that UDA lectin/antibody sandwich ELISA is useful for the quantification of terminal Man-Tf in cerebrospinal fluid.

### <Example 4: Test on amount of terminal Man-Tf in cerebrospinal fluid of various dementia patients>

### (Purpose)

Whether terminal Man-Tf in cerebrospinal fluid would be useful as an index for identification of the contraction of AD and a pathological condition of AD was tested.

### (Method)

The concentration of terminal Man-Tf in cerebrospinal fluid was measured for various dementia cases, i.e., Alzheimer's disease (AD), mild cognitive impairment (MCI), idiopathic normal pressure hydrocephalus (iNPH), and dementia with Lewy bodies/frontotemporal dementia (DLB/FTD), and disease controls, by use of the UDA lectin/antibody sandwich ELISA of Example 3. The concentration was determined through the use of the calibration curve prepared in Example 3 using 15 to 150 ng/mL of a terminal Tri-Man-Tf preparation.

### (Results)

The results are shown in Figure 6. The amount of the terminal Man-Tf contained in the cerebrospinal fluid of the AD patients was 25.543 ± 6.526 µg/mL, which was significantly increased as compared with 20.50 ± 5.41 µg/mL as the amount of the terminal Man-Tf in the disease controls (p = 0.011).

The amount of the terminal Man-Tf in the MCI (AD transition-type MCI according to the present invention) group involving precursor lesions of AD was 27.264 ± 6.105 µg/mL and thus exhibited significant increase (p = 0.002).

The amount of the terminal Man-Tf in other dementia diseases DLB/FTD was measured and was consequently 20.448 ± 5.338 µg/mL. Thus, no significant difference from the disease controls was observed. On the other hand, significant difference from the amount of the terminal Man-Tf in AD was observed (p = 0.003).

The amount of the terminal Man-Tf in iNPH which manifests dementia was 16.892 ± 6.173 µg/mL. Thus, no significant difference from the disease controls was observed. Although iNPH exhibited a decreasing trend of the amount of Man-Tf as compared with that in the disease controls, no significant difference was observed. On the other hand, the amount of the terminal Man-Tf in iNPH exhibited significant difference from the amount of the terminal Man-Tf in AD (p < 0.001). As a result, the AD group was able to be differentiated from the iNPH group with sensitivity of 74.1% and specificity of 90.0%.

Phosphorylated Tau has already been established as a cerebrospinal fluid marker whose level rises in AD, but presents the problem that its level also rises in other tauopathy cases such as FTD. Accordingly, the amount of the terminal Man-Tf was measured only in a FTD group (n = 10) and was consequently 21.0 ± 45.29 µg/mL. This measurement value had no significant difference from the disease controls, but had significant difference from AD (p = 0.014).

These results were obtained by the quantification of the terminal Man-Tf by use of the UDA lectin/antibody sandwich ELISA. In order to test whether or not similar results could also be obtained using other lectins, measurement was also performed by BC2L-A lectin/antibody sandwich ELISA using recombinant BC2L-A lectin which is a terminal mannose binding lectin. The measurement of Man-Tf by ELISA using BC2L-A lectin/antibody was able to differentiate the AD group (12.3 ± 3.9 µg/mL; n = 30) from the iNPH group (6.3 ± 2.6 µg/mL; n = 27) with sensitivity of 96.4% and specificity of 77.8%. This measurement also differentiated the AD group from the MCI group (14.846 ± 7.101 µg/mL: n = 20) with sensitivity of 90.0% and specificity of 81.5% (Figure 8). Also, cases that had been diagnosed as being "normal" at the time of cerebrospinal fluid collection at an outpatient clinic for forgetfulness, but then progressed to MCI, and cases that progressed from MCI to AD (n = 7) exhibited twice the value of the iNPH group (p = 0.001). These results indicated that the level of Man-Tf rises from the early stage of Alzheimer's disease.

Phosphorylated tau protein (p-Tau), a typical diagnostic marker for AD, was measured (n = 28) using the cerebrospinal fluid samples used in the measurement of Man-Tf. As a result of examining the correlation of the p-Tau value with the measurement value of Man-Tf by ELISA using BC2LA/antibody, very strong correlation was shown with r = 0.885 in AD, and strong correlation was also shown with r = 0.729 in MCI (n = 19). The Man-Tf/p-Tau ratio was significantly higher in MCI (231.3 ± 89.8) than in AD (147.8 ± 45.3) (p < 0.001) as shown in Figure 9, indicating that the level of Man-Tf rises earlier than p-Tau. This suggests that Man-Tf correlates with the earlier pathology of AD than that of the neuronal death marker p-Tau.

Likewise, the correlation of the p-Tau value with the measurement value of Man-Tf by ELISA using UDA lectin/antibody was also examined. As a result, strong correlation was shown with r = 0.710 in AD (n = 28). Meanwhile, correlation was also shown with r = 0.576 in MCI (n = 19). The Man-Tf/p-Tau ratio was higher in MCI (474.2 ± 215.2) than in AD (326.9 ± 122.7) (p = 0.011) as shown in Figure 10, indicating that the level of Man-Tf increases earlier (MCI stage) than the level of p-Tau.

From these results, the phenomenon of terminal Man-Tf was considered to be a phenomenon unique to AD and its progression. Also, the increased level of the terminal Man-Tf in MCI suggests that the level of the terminal Man-Tf increases even at the stage of precursor lesions of AD. Thus, the terminal Man-Tf can be expected to be applied to the efficacy evaluation of a therapeutic drug for AD.

Thus, it is considered that the terminal Man-Tf is useful for differentiation from other cases of dementia, particularly, iNPH, and can also serve as an auxiliary diagnostic marker for tauopathy.

### <Example 5: Immunohistochemistry of Alzheimer's disease brain using anti-Tf antibody>

### (Purpose)

It has been suggested that the level of terminal Man-Tf increases as AD progresses. Accordingly, change in the expression of a Tf glycoprotein in the AD brain was analyzed by immunohistochemistry.

### (Method)

A brain tissue of AD was fixed in a phosphate buffer containing 15% formalin, and a paraffin block was then prepared. A sliced section (5 µm) was deparaffinized and then incubated overnight with an anti-Tf polyclonal antibody (DAKO/Agilent Technologies, Inc.; 1:1000) at 4°C. The primary antibody binding site was visualized with Histofine reagent (Nichirei Corp.). Specifically, after washing the section, an enzyme/second antibody-labeled polymer in which peroxidase and Fab' of the second antibody were bound to an amino acid polymer was bound to the primary antibody to perform peroxidase-DAB reaction.

### (Results)

The results are shown in Figure 7. In the AD brain, the anti-human Tf antibody showed an image in which astrocyte-like cells (c) are positive. Also, positive fibers were observed in the neuropils of the first cortical layer (molecular layer) and arranged in bundles, particularly, beneath the pia mater (b). These results are considered to indicate the morbid expression of Tf in the AD brain. Although the glycan isoform of this Tf signal was not determined, these results are considered to be consistent with the increased level of terminal Man-Tf in cerebrospinal fluid.

Connor et al. have reported an image in which "cerebral cortical white matter" astrocytes are positive in AD by immunohistochemistry using an anti-Tf antibody (J Neurosci Res. 1992 Jan; 31 (1): 75-83).

All publications, patents and patent applications cited herein are incorporated herein by reference in their entirety.

## Claims

1. A diagnostic marker for Alzheimer's disease consisting of a transferrin glycoprotein containing a glycan having at least one mannose at a non-reducing end or a transferrin fragment containing the glycan.

2. The diagnostic marker for Alzheimer's disease according to claim 1, wherein the glycan is attached to an asparagine residue at position 432 of transferrin protein consisting of the amino acid sequence represented by SEQ ID NO: 1.

3. The diagnostic marker for Alzheimer's disease according to claim 1 or 2, wherein the diagnostic marker is for the identification of early Alzheimer's disease or Alzheimer's disease transition-type mild cognitive impairment.

4. The diagnostic marker for Alzheimer's disease according to claim 1 or 2, wherein the diagnostic marker is for the differentiation of Alzheimer's disease from other cases of dementia.

5. A diagnostic kit for Alzheimer's disease for detecting a diagnostic marker for Alzheimer's disease according to any one of claims 1 to 4, the diagnostic kit comprising a mannose binding molecule and a transferrin binding molecule.

6. The diagnostic kit according to claim 5, wherein the mannose binding molecule is selected from the group consisting of a mannose binding lectin, an anti-mannose antibody, and an aptamer against mannose.

7. The diagnostic kit according to claim 6, wherein the mannose binding lectin is UDA lectin or BC2L-A lectin.

8. The diagnostic kit according to any one of claims 5 to 7, wherein the transferrin binding molecule is an anti-transferrin antibody and/or an aptamer against transferrin protein.

9. A method for identifying the contraction of Alzheimer's disease, the method comprising:
a measurement step of measuring an amount of a diagnostic marker for Alzheimer's disease according to claim 1 or 2 present in a predetermined amount of a body fluid obtained from each of a test subject and a control group, using a mannose binding molecule and a transferrin binding molecule to obtain measurement values; and
a comparison and identification step of comparing the measurement values of the test subject and the control group obtained in the measurement step to identify the test subject as having Alzheimer's disease when the measurement value of the diagnostic marker for Alzheimer's disease is significantly higher in the test subject.

10. The method for identifying the contraction according to claim 9, wherein the control group is a normal group.

11. The method for identifying the contraction according to claim 9, wherein the control group is an early Alzheimer's disease group.

12. The method for identifying the contraction according to claim 9 or 10, wherein the test subject has a central nervous system disease.

13. A method for identifying Alzheimer's disease transition-type mild cognitive impairment, the method comprising:
a measurement step of measuring an amount of a diagnostic marker for Alzheimer's disease according to claim 1 or 2 present in a predetermined amount of a body fluid obtained from each of a test subject and a control group, using a mannose binding molecule and a transferrin binding molecule to obtain measurement values; and
a comparison and identification step of comparing the measurement values of the diagnostic marker for Alzheimer's disease in the test subject and the control group obtained in the measurement step to identify the test subject as having Alzheimer's disease transition-type mild cognitive impairment when the measurement value of the diagnostic marker for Alzheimer's disease is significantly higher in the test subject, wherein
the test subject has mild cognitive impairment.
